# EUROPEAN PATENT SPECIFICATION

(11) **EP 1 078 601 B1**
(45) Date of publication and mention of the grant of the patent: **04.10.2006**
(21) Application number: 00118072.8
(22) Date of filing: 23.08.2000
(51) Int. Cl.: A61B 17/12, A61F 2/00

(54) **Kit for lung volume reduction**
Set zur Verminderung des Lungenvolumens
Kit destiné a la réduction du volume des poumons

(30) Priority: 24.08.1999 US 379973; 23.03.2000 US 534244
(43) Date of publication of application: 28.02.2001
(73) Proprietor: Spiration, Inc., Redmond, WA 98052 (US)
(72) Inventor: Alferness, Clifton A., Redmond, Washington 98053 (US); Lin, Richard Y., Redwood City, California 94061 (US); Jaeger, Wilfred E., Portoia Valley, California 94028 (US); Gonzalez, Hugo X., Woodinville, Washington 98072 (US); Muff, Diane M., Bellevue, Washington 98008 (US); Sirokman, William A., Kirkland, Washington 98033 (US)
(74) Representative: TBK-Patent

(56) References cited:
- WO-A-98/01084
- US-A- 5 398 844
- US-A- 5 702 343

## Description

The present invention is generally directed to a kit for treating Chronic Obstructive Pulmonary Disease (COPD). The present invention is more particularly directed to a kit comprising a device which may be implanted in the human body to provide lung size reduction by constricting at least a portion of a lung and a form for deploying the device.

Chronic Obstructive Pulmonary Disease (COPD) has become a major cause of morbidity and mortality in the United States over the last three decades. COPD is characterized by the presence of airflow obstruction due to chronic bronchitis or emphysema. The airflow obstruction in COPD is due largely to structural abnormalities in the smaller airways. Important causes are inflammation, fibrosis, goblet cell metaplasia , and smooth muscle hypertrophy in terminal bronchioles.

The incidence, prevalence, and health-related costs of COPD are on the rise. Mortality due to COPD is also on the rise. In 1991 COPD was the fourth leading cause of death in the United States and had increased 33% since 1979.

COPD affects the patient's whole life. It has three main symptoms: cough; breathlessness; and wheeze. At first, breathlessness may be noticed when running for a bus, digging in the garden, or walking up hill. Later, it may be noticed when simply walking in the kitchen. Overtime, it may occur with less and less effort until it is present all of the time.

COPD is a progressive disease and currently has no cure. Current treatments for COPD include the prevention of further respiratory damage, pharmacotherapy, and surgery. Each is discussed below.

The prevention of further respiratory damage entails the adoption of a healthy lifestyle. Smoking cessation is believed to be the single most important therapeutic intervention. However, regular exercise and weight control are also important. Patients whose symptoms restrict their daily activities or who otherwise have an impaired quality of life may require a pulmonary rehabilitation program including ventilatory muscle training and breathing retraining. Long-term oxygen therapy may also become necessary.

Pharmacotherapy may include bronchodilator therapy to open up the airways as much as possible or inhaled ∃-agonists. For those patients who respond poorly to the foregoing or who have persistent symptoms, Ipratropium bromide may be indicated. Further, courses of steroids, such as corticosterocds, may be required. Lastly, antibiotics may be required to prevent infections and influenza and pheumococcal vaccines may be routinely administered. Unfortunately, there is no evidence that early, regular use of pharmacotherapy will alter the progression of COPD.

About 40 years ago, it was first postulated that the tethering force that tends to keep the intrathoracic airways open was lost in emphysema and that by surgically removing the most affected parts of the lungs, the force could be partially restored. Although the surgery was deemed promising, the procedure was abandoned.

The lung volume reduction surgery (LVRS) was later revived. In the early 1990's, hundreds of patients underwent the procedure. However, the procedure has fallen out of favor due to the fact that Medicare stopped remitting for LVRS. Unfortunately, data is relatively scarce and many factors conspire to make what data exists difficult to interpret. The procedure is currently under review in a controlled clinical trial. However, what data does exist tends to indicate that patients benefited from the procedure in terms of an increase in forced expiratory volume, a decrease in total lung capacity, and a significant improvement in lung function, dyspnea, and quality of life.

Improvements in pulmonary function after LVRS have been attributed to at least four possible mechanisms. These include enhanced elastic recoil, correction of ventilation/perfusion mismatch, improved efficiency of respiratory musculature, and improved right ventricular filling.

Lastly, lung tranplantation is also an option. Today, COPD is the most common diagnosis for which lung transplantation is considered. Unfortunately, this consideration is given for only those with advanced COPD. Given the limited availability of donor organs, lung transplant is far from being available to all patients.

In view of the foregoing, there is a need in the art for a new and improved therapy for COPD. More specifically, there is a need for such a therapy which provides more permanent results than pharmacotherapy while being less traumatic than LVRS. The present invention is directed to device which provides such an improved therapy for COPD.

The improvements in pulmonary function resulting from LVRS cannot be ignored. However, the surgery is very invasive and fraught with complications. Among the complications is the potential for lung air leaks. Lung tissue is very thin, and fragile hence difficult to suture together. After a lung portion is sectioned and removed, the remaining lung is most often restructured with suture staples. In about thirty percent (30%) of the cases, the difficulty with suturing lung tissue results in air leaks. Treatment for such air leaks depends upon their severity and often, in the most serious cases, requires further open chest surgery.

According to the present invention, the need for an improved therapy for COPD is met by a kit as set forth in claim 1. The kit includes a device comprising a jacket of flexible material configured to cover a portion of a lung and to constrict the lung portion.

The use of a jacket of flexible material is known from other fields of therapy. For example, WO-A-98-01084 is directed to an device for treating pulmonary emphysema, comprising a sheath of elastic material that is applied to an entire lung. The sheath includes only a single opening and is applied to confine the lung after complete lysis of pleural adhesions and the section of pulmonary ligament up to the lower pulmonary vein to completely free the mediastinal, diaphragmatic and costal surfaces of the lung. This permits the sheath to be oriented with its upper portion around the apical area of the lung after the whole lung is passed through the opening. The lung is then ventilated. Once applied, the sheath merely confines the lung. With the proper size sheath and after ventilation, the lung should reach the normal theoretical values of the pulmonary capacity of an individual on which a pressure of 10 to 15 cm of water is exerted. This prevents the lung from expanding and collapsing.

The U.S. Patent No. 5,702,343 is directed to a cardiac reinforcement device or support jacket that limits the outward expansion of a heart wall during diastolic chamber filling beyond a predetermined size. The size-adjustable jacket is applied over a heart to confine and preclude expansion of the heart as a therapy for ventricular dilation. The degree of expansion constraint applied to the heart is predetermined by the physician based on, for example, cardiac output performance or cardiac volume. The cardiac reinforcement device provides cardiac reinforcement that constrains cardiac expansion during diastolic filling of the heart to a predetermined size, but does not impair systolic function.

The U.S. Patent No. 5,398,844 is directed to a dispenser for carrying and dispensing a plurality of ligating bands in sequence at discrete sites. The dispenser includes a support having a cylindrical outer surface that carries the plurality of spaced apart, discrete ligating bands in a stretched condition and ordered serially on the cylinder of the support. Each ligating band has a displacement structure in the form of a filament connected to it. The filament, when pulled, displaces its attached ligating band from the cylindrical outer surface of the support and releases the single stretched ligating band to constrict tissue. The ligating bands have a structure substantially in the shape of a ring, they are slid off the dispenser one at a time, and over the targeted tissue, stopping blood circulation in or through the target tissue and causing it to die.

As will be apparent from the detailed description, the present invention further provides a kit including device for suppressing air leaks in lung tissue. Air leaks in lungs can be caused by surgery and other causes. With increasing age, a patient may develop a weakened section of lung which may then rupture due to an extreme pressure differential, such as may result from simply a hard sneeze. AIDS patients can suffer from air leaks in their lungs. Air leaks in lungs can further be caused by a puncture from a broken rib or a stab wound. The air leak suppression, in accordance with the present invention, does not require any suturing of the effected lung tissue. Still further, by constricting a large enough portion of a lung in accordance with the present invention, lung volume reduction with the concomitant improved pulmonary function may be obtained without the need for any suturing of lung tissue at all.

The detailed description will be given in connection with preferred embodiments of the invention and with reference to the accompanying drawings.
**FIG. 1** is a simplified sectional view of a thorax illustrating a healthy respiratory system.
**FIG. 2** is a sectional view similar to **FIG. 1** but illustrating a respiratory system suffering from COPD.
**FIG. 3** is a sectional view similar to **FIG. 1** but illustrating a respiratory system suffering from an air leak in a lung lobe.
**FIG. 4** is a sectional view illustrating the lung lobe having the air leak in a deflated condition due to the air leak.
**FIG. 5** is a sectional view of the respiratory system of **FIG. 3** with a lung constriction device embodying the present invention being disposed over a lung portion to be constricted for suppressing the air leak.
**FIG. 6** is a sectional view illustrating the lung constricting device constricting the effected lung portion and suppressing the air leak.
**FIG. 7** illustrates a lung constriction device embodying the present invention and a form which may be used in a mechanical method for deploying the constriction device.
**FIG. 8** illustrates an initial step in practicing the mechanical method of deployment.
**FIG**. **9** illustrates a further step in the mechanical deployment of the constriction device.
**FIG. 10** illustrates the step of pulling the lung portion to be constricted into the constriction device in accordance with the mechanical method embodiment.
**FIG. 11** illustrates the manner in which an expansion force may be released from the constriction device as a final step in deploying the constriction device in accordance with the mechanical method embodiment.
**FIG. 12** illustrates the constriction device fully deployed as a result of the mechanical method embodiment illustrated in **FIGS. 7- 11.**
**FIG. 13** illustrates an initial step of a further method of deploying the lung constriction device.
**FIG. 14** illustrates an intermediate step in the further method embodiment of deploying the lung constriction device.
**FIG**. **15** illustrates a final step in the further method embodiment of deploying the lung constriction device.
**FIG. 16** illustrates an initial step of a still further method of deploying the lung constriction device.
**FIG. 17** illustrates an intermediate step in the still further method embodiment of deploying the lung constriction device.
**FIG. 18** illustrates a final step in the still further method embodiment of deploying the lung constriction device.
**FIG. 19** is a sectional view illustrating the lung constricting device constricting a lung portion to be sectioned for lung volume reduction.
**FIG. 20** illustrates the lung portion after being sectioned in accordance with a further embodiment of the present invention.

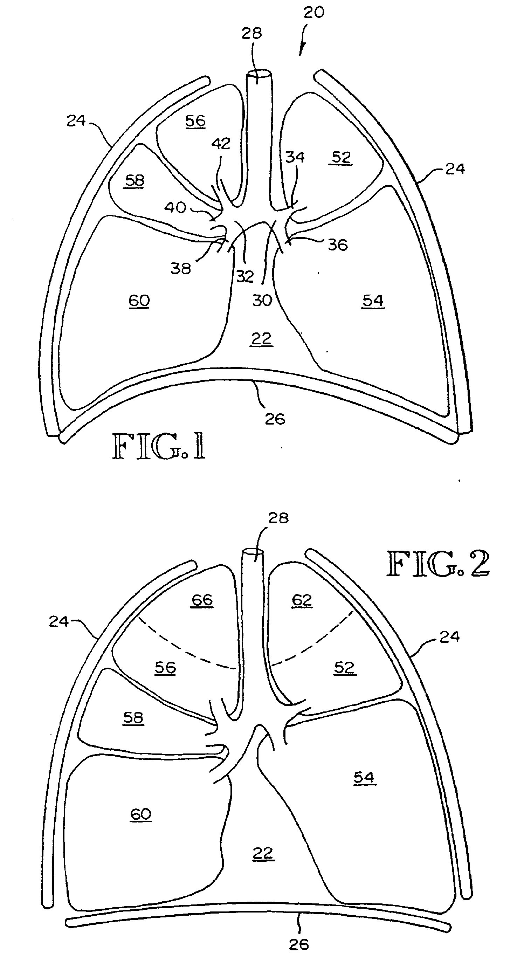

Referring now to **FIG. 1**, it is a sectional view of a healthy respiratory system. The respiratory system **20** resides within the thorax **22** which occupies a space defined by the chest wall **24** and the diaphragm **26**.

The respiratory system **20** includes the trachea **28**, the left mainstem bronchus **30**, the right mainstem bronchus **32**, and the bronchial branches **34, 36**, **38, 40**, and **42**. The respiratory system **20** further includes left lung lobes **52** and **54** and right lung lobes **56, 58**, and **60**. Each bronchial branch communicates with a respective different portion of a lung lobe, either the entire lung lobe or a portion thereof.

Characteristic of a healthy respiratory system is the arched or inwardly arcuate diaphragm **26**. As the individual inhales, the diaphragm **26** straightens to increase the volume of the thorax **22**. This causes a negative pressure within the thorax. The negative pressure within the thorax in turn causes the lung lobes to fill with air. When the individual exhales, the diaphragm returns to its original arched condition to decrease the volume of the thorax. The decreased volume of the thorax causes a positive pressure within the thorax which in turn causes exhalation of the lung lobes.

In contrast to the healthy respiratory system of **FIG. 1, FIG. 2** illustrates a respiratory system suffering from COPD. Here it may be seen that the lung lobes **52, 54, 56, 58**, and **60** are enlarged and that the diaphragm **26** is not arched but substantially straight. Hence, this individual is incapable of breathing normally by moving the diaphragm **28**. Instead, in order to create the negative pressure in the thorax **22** required for breathing, this individual must move the chest wall outwardly to increase the volume of the thorax. This results in inefficient breathing causing these individuals to breathe rapidly with shallow breaths.

It has been found that the apex portion **62** and **66** of the upper lung lobes **52** and **56**, respectively, are most affected by COPD. Hence, the preferred embodiment will be described for treating the apex **66** of the right, upper lung lobe **56**. However, as will be appreciated by those skilled in the art, the present invention may be applied to any lung portion without departing from the present invention.

The kit of the present invention treats COPD by deriving the benefits of lung volume reduction surgery without the need of performing lung volume reduction surgery. As will be seen hereinafter, aspects of the present invention contemplate permanent collapse of a lung portion or lung portions most affected. This leaves extra volume within the thorax for the diaphragm to assume its arched state for acting upon the remaining healthier lung tissue. As previously mentioned, this should result in improved pulmonary function due to enhanced elastic recoil, correction of ventilation/perfusion mismatch, improved efficiency of respiratory musculature, and improved right ventricle filling.

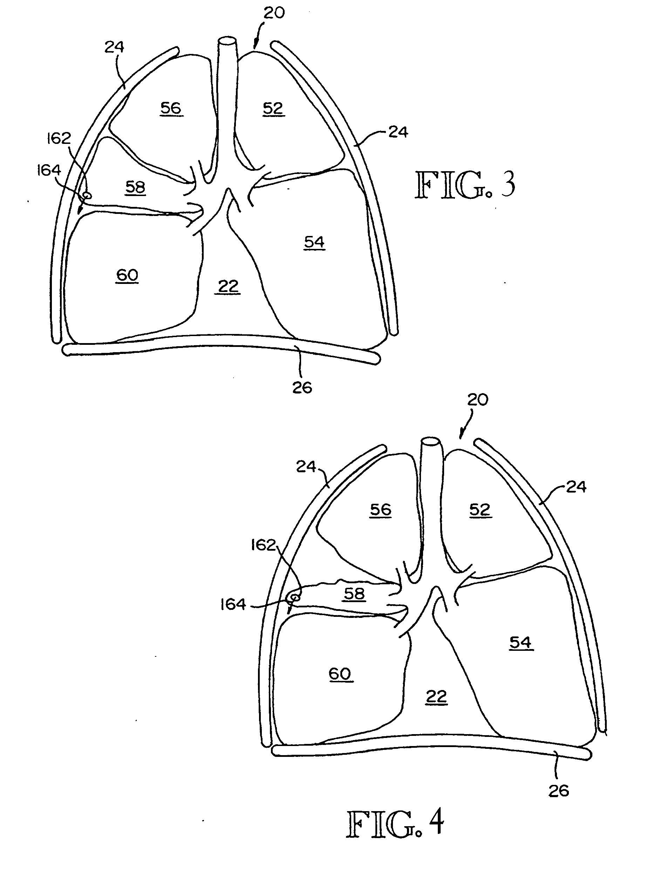

**FIG**. **3** illustrates the respiratory system **20** just after suffering an air leak or rupture. Here it may be seen that the rupture **162** has occurred in lung lobe **58**. As a result, air is escaping from the lung lobe **58** as indicated by the arrow **164**. Hence, this individual is incapable of breathing normally. The negative pressure created by the moving diaphragm **26** causes some of the air taken into lobe **58** to be lost through the rupture **162**. When the diaphragm **26** returns to its arched configuration, the positive pressure produced thereby forces still more air from lobe **58** through the rupture. Eventually, within a short time, the lobe **58** collapses as illustrated in **FIG. 4** and becomes nonfunctional to support respiration.

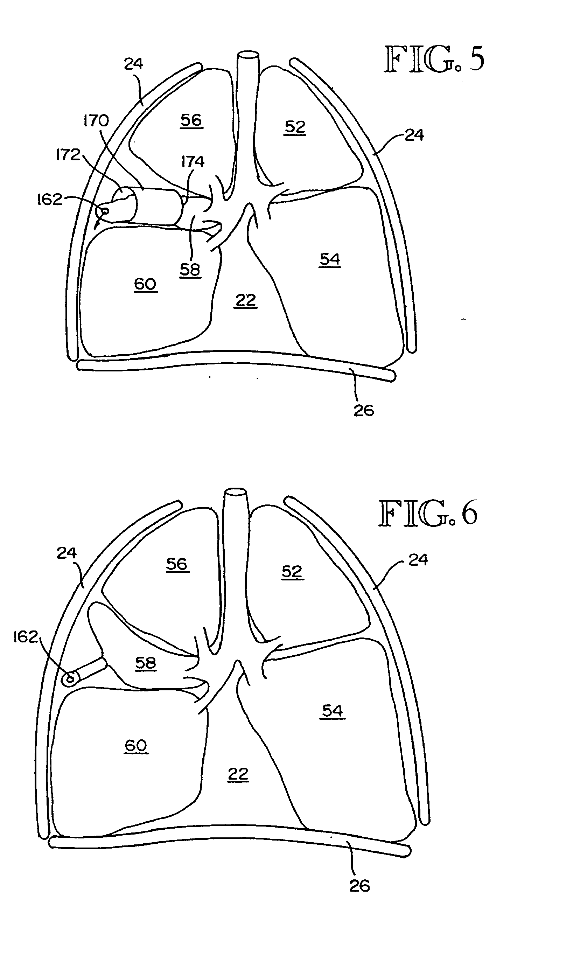

**FIG. 5** shows a lung constriction device **170** embodying the present invention in the process of being deployed on the effected lung lobe **58**. The device **170** is configured as a jacket formed of a sheet or flexible fabric of biocompatible material. The material may be both flexible and expandable material formed from silicone rubber, polyurethane, expanded polytetraflouroethylene, polyester and polyurethane, or nylon and polyurethane, for example. The jacket may more specifically be formed from a sheet or fabric of 70% nylon and 30% polyurethane. The jacket is preferably opened at both ends **172** and **174** and, as illustrated, may be generally cylindrical in configuration.

In accordance with one embodiment, the jacket is applied to the portion of the lung lobe having the leak or puncture while the jacket is in an expanded condition. This may be accomplished, as will be seen hereinafter, by expanding the jacket and then pulling the lung portion into the jacket. When the effected lung portion is thus disposed with respect to the jacket as illustrated in **FIG. 5**, the expansion of the device is released as seen, for example, in **FIG. 6.** With the expansion released, the jacket is permitted to contract or collapse about the lung portion to constrict the lung portion and effectively suppress the leak or puncture.

When the lung portion is thus constricted, the air leakage will be suppressed. The lung lobe **58** thereafter, during successive breaths, will reinflate and become functional once again to support respiration.

The use of the device **170** need not be restricted to the suppression of air leakages in lungs. It may, for example, find use to advantage in constricting a lung portion suffering from COPD to simulate or achieve lung volume reduction. All of the beneficial effects of lung volume reduction surgery may be realized and, most importantly, without requiring suturing of lung tissue.

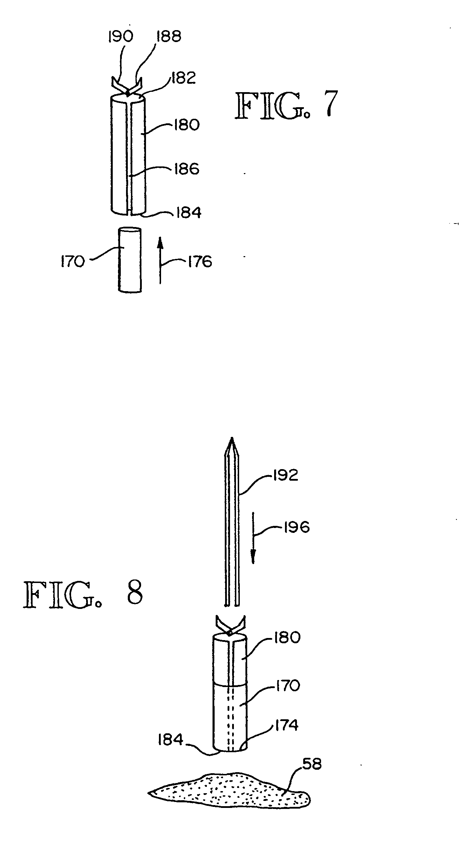

**FIGS**. **7 - 12** illustrate a mechanical process for deploying the lung constriction device **170**. In an initial step, as illustrated in **FIG. 7**, the device **170** is first aligned with an expansion mandrel or form **180**. The device **170** is then moved towards the form **180** as indicated by the arrow **176**.

In accordance with this embodiment, the form **180** is hollow, has opened ends **182** and **184** and has a configuration similar to that of the device **170**. In addition, the form has a longitudinal slit **186** rendering the form expandable in a transverse direction. The form further includes tabs **188** and **190** which, when pressed towards each other, cause the form to expand in the transverse direction.

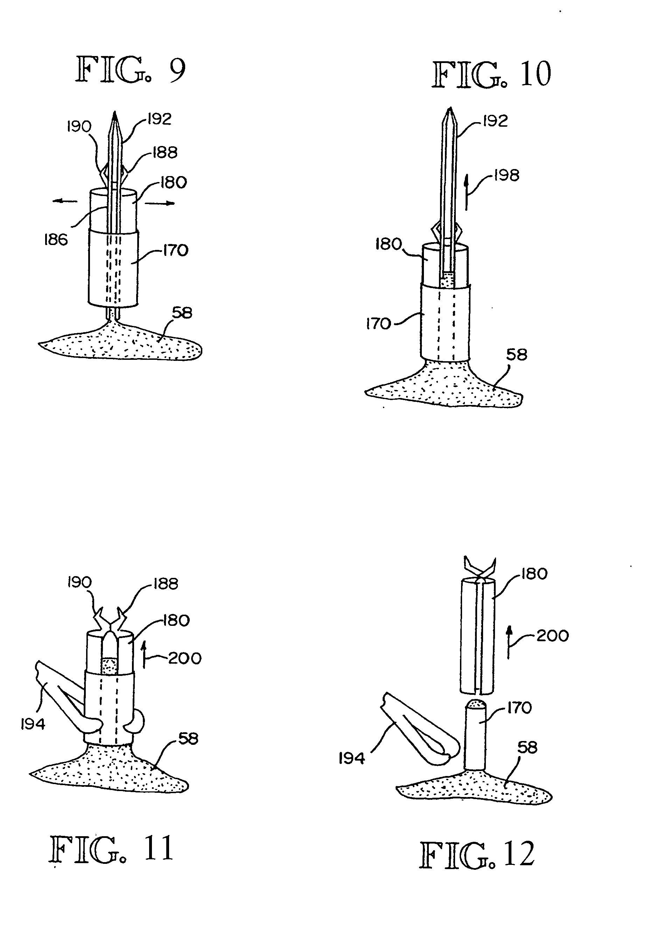

The device **170** is applied to the form **180** until the end **174** of the device **170** is at the end **184** of the form **180** as illustrated in **FIG. 8**. An atraumatic instrument, such as a forceps **142**, is then aligned with the form **180** and moved relative thereto through the form in the direction of arrow **196** and into engagement with the lung tissue **58** as illustrated in **FIG. 9.**

The forceps **192** are then used to grab the lung tissue **58**. Then, the tabs **188** and **190** of the form **180** are pressed toward each other to cause the form **180** to expand in a transverse direction. This may be noticed by the longitudinal slit **186** becoming noticeably wider. The expansion of the form **180** in the transverse direction imparts an expansion force on the device **170**, causing it to similarly expand to an expanded condition. With the device **170** thus expanded, the forceps are then retracted as illustrated in **FIG. 10** in the direction of arrow **198**, to pull the lung tissue into the form **180** and device **170**. Preferably, although not necessarily, the lung tissue is pulled until it extends entirely through the device **170**.

The process continues as illustrated in **FIG. 11.** Here, the tabs **188** and **190** are released. Given the volume of lung tissue within the form **180** and device **170**, the device **170** remains in an expanded condition. Now, a suitable instrument **194** is used to hold the device **170** in place while the form **180** is moved in the direction of the arrow **200** to withdraw the form **180** from the device **170**.

As illustrated in **FIG. 12,** the process is completed when the form **180** is totally withdrawn from the device **170**. In doing so, the expansion force applied to the device **170** by the form **180** is released, permitting the device **170** to collapse or contract about the lung tissue **58** drawn into the device **170**. The device **170** now constricts the lung tissue to effect air leak suppression or lung volume reduction, for example.

Alternatively, the form **180** need not be expandable if the device **170** is not expandable. Here, the process of pulling the lung tissue into the mandrel **180** and device **170** will cause the lung tissue to collapse. With the device **170** being dimensioned for constricting the lung tissue, once the mandrel is removed, the lung tissue will remain in and be constricted by the device **170** as illustrated in **FIG. 12**.

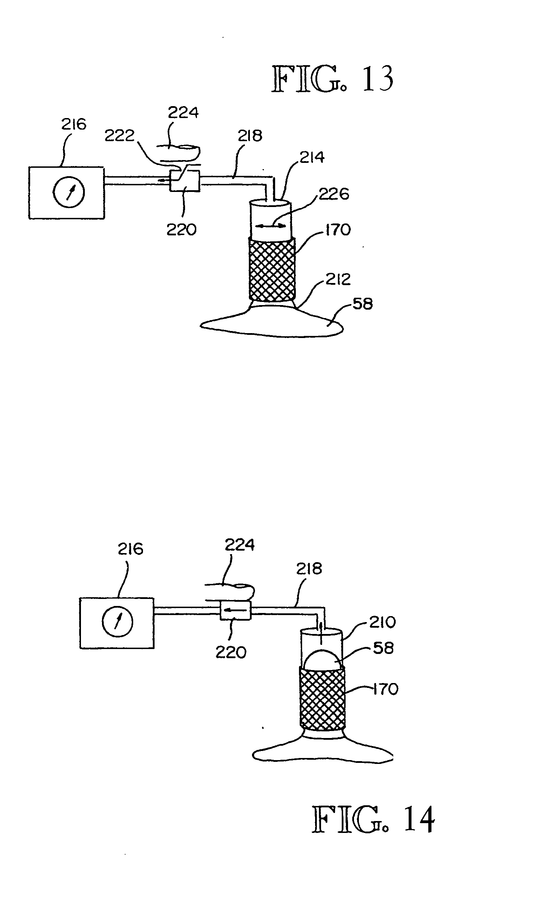

**FIGS. 13-15** illustrate another embodiment of deploying the lung constriction device **170.** Here, rather than using mechanical pulling of the lung tissue into the device **170**, vacuum pressure is utilized instead for pulling the lung tissue into the device **170**. This permits the procedure to be more automated and potentially less traumatic to the lung tissue being constricted.

As will be noted in **FIG. 13**, the mandrel or form **210** takes the form of a cylinder having an opened end **212** and a closed end **214**. The closed end **214** is coupled to a vacuum source **216** through a conduit **218** and a valve **220**. The valve **220** has an aperture **222** which, when closed by, for example, a finger **224**, causes the vacuum to be pulled through the conduit **218** and form **210**. As illustrated in **FIG. 13**, the valve is in an opened condition.

The form **210** has a diameter dimension **226** which is substantially greater than the diameter dimension of the device **170** when the device is expandable and in a nonexpanded condition. As seen in **FIG. 13**, the device **170** has been applied over the form **210** so that the form imparts an expansion force to the device **170**. The opened end **212** of the form **210** is in contact with the lung tissue **58** to be constricted.

Referring now to **FIG. 14**, the finger **224** has now closed the valve **220**. The vacuum is now being pulled through the conduit **218** and form **210**. This causes the lung tissue **58** to be pulled into the form **210** and the device **170** while the device **170** is in an expanded condition.

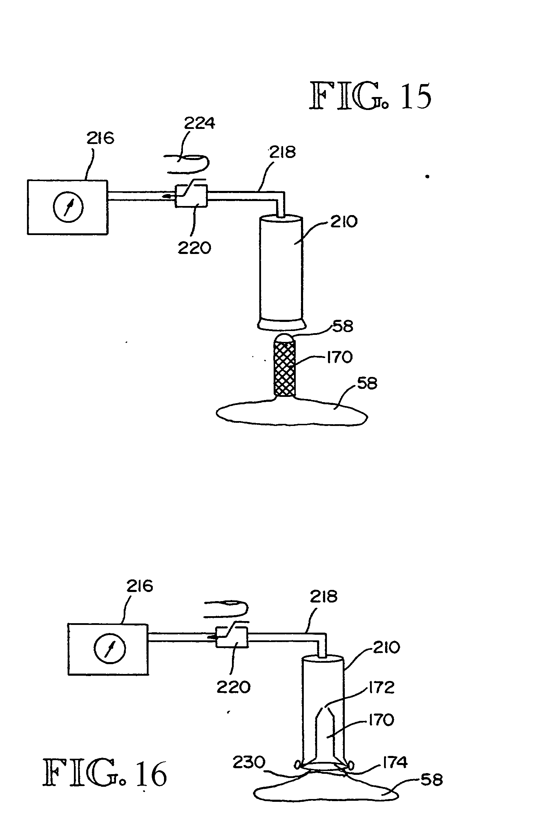

After the lung tissue **58** has been pulled into the form **210** and the device **170**, the device may be held in position and the form **210** withdrawn from the device **170** and the lung tissue **58**. When this is completed, as best seen in **FIG. 15**, the vacuum suction may be released by opening the valve **220**. More importantly, the expansion force of the form **210** on the device **170** is released to permit the device **170** to collapse or contract about the lung tissue **58**. The device **170** is now deployed for constricting the lung tissue and providing leak suppression or lung volume reduction, for example.

**FIGS. 16** - **18** illustrate a further embodiment of deploying the lung constriction device **170**. Here again, a vacuum suction is utilized for pulling the lung tissue into the device **170.**

As illustrated in FIG. 16, the vacuum source **216**, the conduit **218**, and the valve **220** are again used to establish the vacuum suction in the form **210**. Here, however, the device **170** is positioned inside of the form **210** with the end **174** of the device **170** being stretched and held by the lip **230** of the form **210**. As a result, when the valve **220** is closed, the vacuum is pulled through the mandrel **210** and the device **170** due to the opened end **172** of the device **170**.

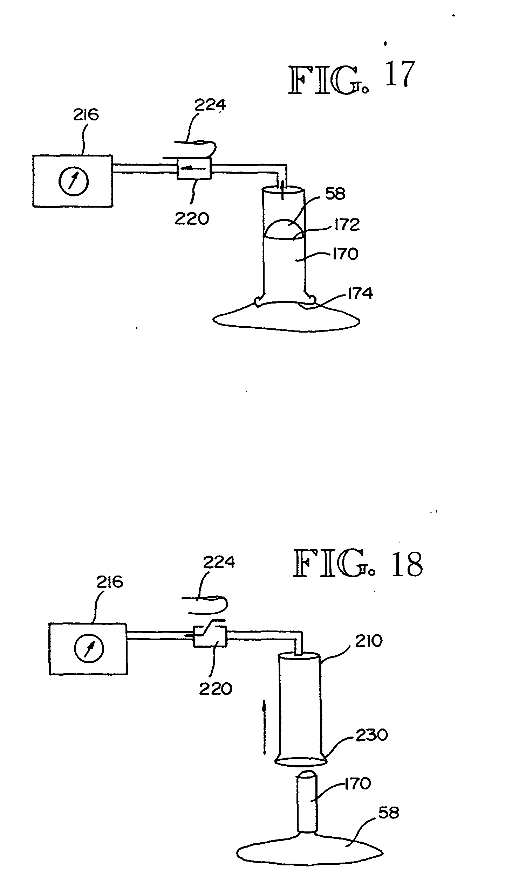

Now, when the lung tissue **58** is brought into engagement with the end **174** of the device **170** and the vacuum is pulled with the closure of valve **220**, the lung tissue is pulled directly into the device **170** as illustrated in FIG. 17. The vacuum is pulled until the lung tissue **58** to be constricted preferably extends entirely through the device **170** past the end **172**. As will be further noted, the lung tissue itself exerts an expansion force on the device **170** as the lung tissue is pulled into the device **170**.

After the lung tissue **58** has been pulled into the device **170**, the end **174** of the device **170** may be released from the lip **230** of the form **210** to permit the form **210** to be withdrawn from the device **170**. When this is completed, as best seen in FIG. 18, the vacuum suction may be released by opening the valve **220**. The release of the vacuum also releases the expansion force on the device **170**. With the expansion force released, the device is permitted to collapse or contract about the lung tissue 58. The device **170** is now deployed for constricting the lung tissue and providing leak suppression or lung volume reduction, for example.

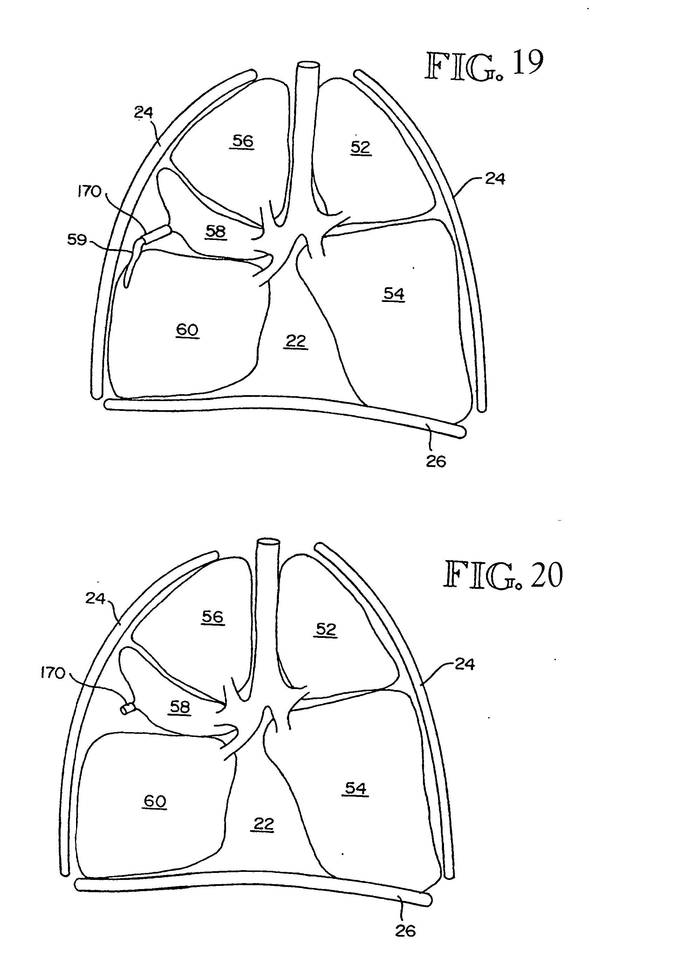

Referring now to FIG. 19, it illustrates a manner in which the lung constriction device **170** may be employed for effecting lung volume reduction to a greater extent. In accordance with this embodiment, the lung portion **59** of lobe **58** has been pulled through the device **170** and is being constricted by the device **170**. The device **170** and the manner of pulling the lung portion **59** therethrough may conform to any of the embodiments previously described herein.

In accordance with this embodiment, the device **170** is formed of severable material, such as, any of the materials previously described. This enables the device or jacket **170** to be severed or cut intermediate its ends as illustrated in FIG. **20** to section the lung portion 59. The portion of the device **170** remaining on the lobe **58** continues to constrict the lung tissue therein to form an effective seal from leakage. Hence, in accordance with this embodiment of the present invention, lung volume reduction is rendered an available treatment while negating the need of conventional lung sectioning and suturing thus avoiding the potentially severe complications which accompany those procedures.

## Claims

1. A kit comprising a lung constricting device (170) for covering a lung portion (58) of a lung to constrict the lung portion (58), and a form (180; 210) for deploying the device (170), wherein
the device (170) comprises a jacket of flexible and expandable material, the jacket being generally cylindrical in configuration with its length greater than its width and having a pair of open ends (172,174); and
the form (180; 210) has a configuration similar to that of the device (170) to import a transverse expansion force to the device (170) to bring it into an expanded condition so that lung tissue of the lung portion (58) may be pulled into the device (170) in the expanded condition.

2. A kit according to claim 1, wherein the jacket is formed of a severable material to permit the device to be severed or cut intermediate its ends (172, 174).

3. A kit according to claim 2, wherein the device (170) is configured to be severed or cut on the lung to section the lung portion (58).

4. A kit according to any of claims 1 to 3, wherein the jacket is formed of an expandable mesh material.

5. A kit according to claim 4, wherein the device is contractible about the lung portion upon release of the expansion force.

6. A kit according to any of claims 1 to 5, wherein the jacket is formed of a sheet of biocompatible material.

7. A kit according to any of claims 1 to 6, wherein the jacket is formed of one of silicone rubber, polyurethane, expanded polytetrafluoroethylene, polyester and polyurethane, nylon and polyurethane.

8. A kit according to any of claims 1 to 7, wherein the form (180) comprises a hollow cylinder with open ends (182, 184) and has a longitudinal slit (186), so that, in use, the lung tissue can be pulled into the form (180) and the device (170) can be withdrawn from the form (180) to constrict the lung tissue.

9. A kit according to any of claims 1 to 7, wherein the form (210) comprises a hollow cylinder with an open end (212) and a closed end (214), said closed end being for coupling to a vacuum source to cause, in use, the lung tissue to be pulled into the form (210).

## Patentansprüche

1. Set aus einer Lungeneinengungsvorrichtung (170) zum Bedecken eines Lungenabschnitts (58) einer Lunge, um den Lungenabschnitt (58) einzuengen, und einer Form (180; 210) zum Ausbringen der Vorrichtung (170), wobei
die Vorrichtung (170) einen Mantel aus flexiblem und dehnbarem Material umfasst, der eine allgemein zylinderförmige Gestaltung, bei der seine Länge größer als seine Breite ist, und ein Paar offener Enden (172, 174) hat; und
die Form (180; 210) eine ähnliche Gestaltung wie die Vorrichtung (170) hat, die der Vorrichtung (170) eine Querdehnungskraft mitgibt, um sie in einen ausgedehnten Zustand zu bringen, so dass im ausgedehnten Zustand Lungengewebe des Lungenabschnitts (58) in die Vorrichtung (170) gezogen werden kann.

2. Set nach Anspruch 1, bei dem der Mantel aus einem trennbaren Material gebildet ist, damit die Vorrichtung zwischen ihren Enden (172, 174) durchtrennt oder durchschnitten werden kann.

3. Set nach Anspruch 2, bei der die Vorrichtung (170) so gestaltet ist, dass sie auf der Lunge durchtrennt oder durchschnitten werden kann, um den Lungenabschnitt (58) zu durchschneiden.

4. Set nach einem der Ansprüche 1 bis 3, bei dem der Mantel aus einem dehnbaren Netzmaterial gebildet ist.

5. Set nach Anspruch 4, bei dem sich die Vorrichtung beim Freiwerden der Dehnkraft um den Lungenabschnitt zusammenziehen kann.

6. Set nach einem der Ansprüche 1 bis 5, bei dem der Mantel aus einer dünnen Lage biokompatiblen Materials gebildet ist.

7. Set nach einem der Ansprüche 1 bis 6, bei dem der Mantel aus entweder Silikongummi, Polyurethan, Polytetrafluorethylenschaum, Polyester und Polyurethan oder Nylon und Polyurethan gebildet ist.

8. Set nach einem der Ansprüche 3 bis 7, bei dem die Form (180) einen Hohlzylinder mit offenen Enden (182, 184) umfasst und einen Längsschlitz (186) hat, so dass das Lungengewebe im Einsatz in die Form (180) hineingezogen werden kann und die Vorrichtung (170) von der Form (180) weggezogen werden kann, um das Lungengewebe einzuengen.

9. Set nach einem der Ansprüche 1 bis 7, bei dem die Form (210) einen Hohlzylinder mit einem offenen Ende (212) und einem geschlossenen Ende (214) umfasst, wobei das geschlossene Ende zum Ankoppeln einer Vakuumquelle dient, um im Einsatz zu bewirken, dass das Lungengewebe in die Form (210) gezogen wird.

## Revendications

1. Kit comprenant un dispositif de constriction du poumon (170) pour couvrir une partie de poumon (58) d'un poumon pour comprimer la partie de poumon (58), et une forme (180 ; 210) pour déployer le dispositif (170), dans lequel
le dispositif (170) comprend une enveloppe en matériau flexible et extensible, l'enveloppe étant en règle générale cylindrique en configuration avec sa longueur plus grande que sa largeur et ayant une paire d'extrémités ouvertes (172, 174) ; et
la forme (180 ; 210) présente une configuration semblable à celle du dispositif (170) pour conférer une force d'expansion transversale au dispositif (170) pour le porter dans un état dilaté de sorte que le tissu pulmonaire de la partie de poumon (58) puisse être tiré dans le dispositif (170) à l'état dilaté.

2. Kit selon la revendication 1, dans lequel l'enveloppe est formée d'un élément séparable pour permettre au dispositif d'être séparé ou coupé à un niveau intermédiaire de ses extrémités (172, 174).

3. Kit selon la revendication 2, dans lequel le dispositif (170) est configuré pour être séparé ou coupé sur le poumon pour sectionner la partie de poumon (58).

4. Kit selon l'une quelconque des revendications 1 à 3, dans lequel l'enveloppe est formée d'un matériau en filet extensible.

5. Kit selon la revendication 4, dans lequel le dispositif est capable d'être contracté autour de la partie de poumon à la libération de la force d'expansion.

6. Kit selon l'une quelconque des revendications 1 à 5, dans lequel l'enveloppe est formée d'une feuille de matériau biocompatible.

7. Kit selon l'une quelconque des revendications 1 à 6, dans lequel l'enveloppe est formée d'un caoutchouc de silicone, polyuréthane, polytétrafluoroéthylène expansé, polyester et polyuréthane, nylon et polyuréthane.

8. Kit selon l'une quelconque des revendications 1 à 7, dans lequel la forme (180) comprend un cylindre creux avec des extrémités ouvertes (182, 184) et présente une fente longitudinale (186), de sorte que, à l'utilisation, le tissu pulmonaire puisse être tiré dans la forme (180) et que le dispositif (170) puisse être retiré de la forme (180) pour étrangler le tissu pulmonaire.

9. Kit selon l'une quelconque des revendications 1 à 7, dans lequel la forme (210) comprend un cylindre creux avec une extrémité ouverte (212) et une extrémité fermée (214), ladite extrémité fermée étant à coupler à une source de vide pour faire, à l'utilisation, tirer le tissu pulmonaire dans la forme (210).
